(19) Europäisches Patentamt / European Patent Office / Office européen des brevets

(11) **EP 2 001 386 B1**

(12) **EUROPEAN PATENT SPECIFICATION**

(45) Date of publication and mention
of the grant of the patent:
**02.01.2013 Bulletin 2013/01**

(51) Int Cl.:
*A61B 18/20* (2006.01)  *A61N 7/00* (2006.01)

(21) Application number: **07736158.2**

(22) Date of filing: **29.03.2007**

(86) International application number:
**PCT/IL2007/000418**

(87) International publication number:
**WO 2007/113817 (11.10.2007 Gazette 2007/41)**

(54) **APPARATUS FOR PHOTOTHERMOLYSIS**

GERÄT FÜR DIE PHOTOTHERMOLYSE

APPAREIL DE PHOTOTHERMOLYSE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **06.04.2006 US 789567 P**
**20.07.2006 US 489587**

(43) Date of publication of application:
**17.12.2008 Bulletin 2008/51**

(73) Proprietor: **Lite Touch Ltd.**
**44640 Kfa Sava (IL)**

(72) Inventor: **KORENBERG, Dan**
**89031 Arad (IL)**

(74) Representative: **Lecomte, Didier et al**
**Lecomte & Partners Sàrl**
**P.O. Box 1623**
**1016 Luxembourg (LU)**

(56) References cited:
**WO-A-03/101328**   **WO-A-2006/012752**
**WO-A1-97/47250**   **WO-A1-99/07438**
**US-A1- 2004 230 260**   **US-B1- 6 187 001**
**US-B1- 6 267 771**   **US-B2- 6 629 971**

## Description

The present invention relates to photothermolysis, such as methods and devices for treatment of skin.

BACKGROUND OF THE INVENTION

Treatment of the upper layer of the skin, epidermis and dermis is performed in order to achieve younger and nice appearance of the skin. The treatments are currently widely available by a large number of aesthetic providers. Light therapy was proven in the last two decades to be a very effective tool in addressing a variety of lesions in the skin such as pigmented and vascular lesions, and hair follicles. Both laser light and incoherent light energy have been suggested for use. The basic theory behind the use of pulse optical energy is to create thermal distraction of the selected lesion, without damaging the surrounding tissue. In order to selectively heat the lesion, the optical spectrum, pulse duration, energy density, and spot size have to be carefully set. Existing systems use pulse durations in the range of 2 -100 msec and energy density of 10-60 J/cm$^2$.

Removal of excess or otherwise undesired hair from different parts of the human body has become more and more popular, particularly for cosmetic and aesthetic reasons.

The traditional methods of hair removal such as shaving, use of wax or creams, or mechanical plucking is unsatisfactory because of the need to repeat the treatment every certain period of time.

Therefore, methods for permanently removing the hair have been developed, based on causing irreversible damage to the hair follicles. Most of the presently applied methods are based on thermal destruction of the hair shaft and follicle using either thermolysis or photothermolysis.

The thermolysis process (see for example US Patent 5,891,139) involves the insertion of a current carrying needle into every hair follicle. This procedure is however painful and time consuming.

Another technique for destroying hair follicles is to apply electromagnetic energy in the form of light. Use of light to denature specific kinds of tissue is known as photothermolysis (see e.g. US Patents 5,425,728, 5,683,380, 5,885,273, 6,080,147 and many others). Sources of light include pulsed laser beams or Intense Pulse Light technology (IPL). In contrast to a monochromatic laser light, the IPL has a broad spectrum. In both cases, the light energy is selectively absorbed by the hair shaft, and dissipated at the follicle to create thermal distraction and permanent hair loss. Also in both cases, the light energy must penetrate through the epidermis before it can reach the depth necessary to cause damage to the hair follicles.

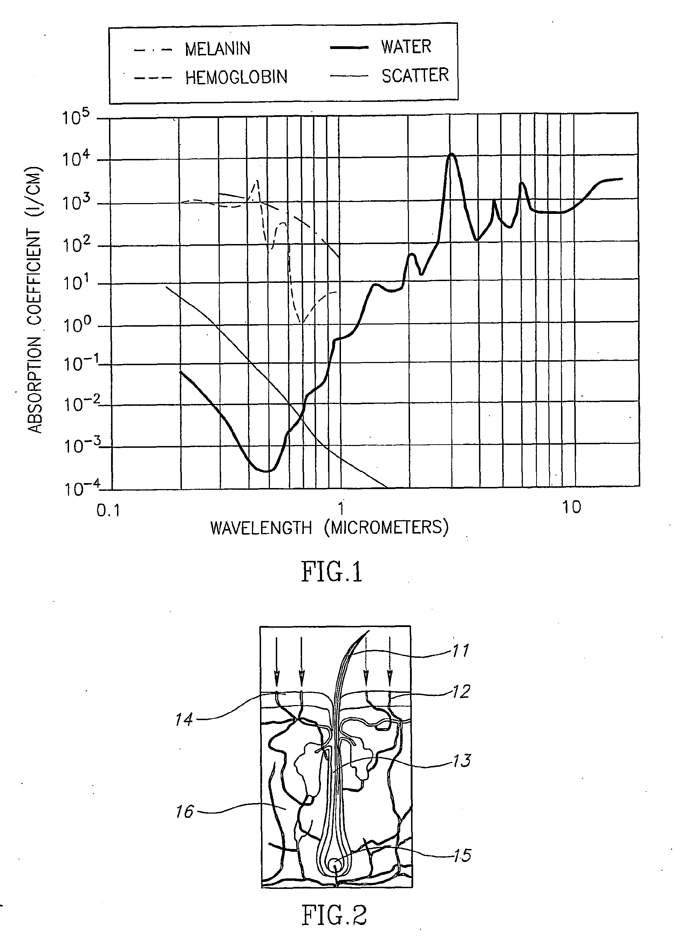

In this context it should be noticed that three major factors govern the efficiency of light/tissue interaction: reflection, absorption, and scattering. A significant portion of light is reflected away from the skin surface. The rest of the light is absorbed by tissue chromophors, which are melanin, contained in the epidermis and in the hair shafts, and hemoglobin, contained in the blood. The chart of Fig. 1 gives the relation between the absorption factor and the wavelength of irradiated light.

US 2004/0230260 A1 discloses an apparatus for photothermolysis comprising an incoherent light source in accordance with the preamble of claim 1.

The physiology of a typical human skin-grown hair is now briefly described with reference to Fig. 2 illustrating a section of human skin with one hair.

The hair comprises a shaft 11 expending above skin surface 12 and hair follicle 13 with the papilla 15 at the bottom of the follicle.. The hair shaft 11 passes through epidermis 14 into dermis 16, with the papilla 15 being about 4 mm below the skin surface 12.

The hair growth cycle includes three stages: anagen, catagen and telogen. In the anagen stage, the papilla 15 and the shaft 11 are connected. The anagen stage is considered to last for the longest period of time among the three stages. In the catagen stage, the papilla 15 starts to break away from the shaft 11. In the telogen stage, which is much shorter than the other stages, the papilla 15 is completely disconnected from the shaft 11.

It is believed that for preventing re-growth of hair both the papilla 15 and the shaft 11 should be destroyed. The stage during which the papilla 15 is most influenced by light energy is the anagen stage.

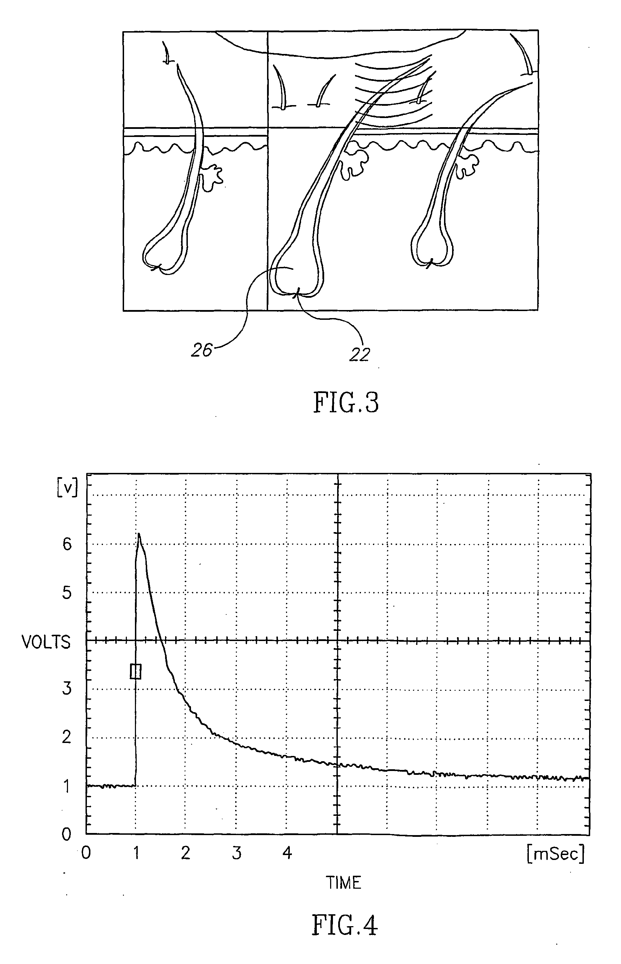

The destruction of hair follicle 13 by light energy is illustrated in Fig. 3. Application of the pulsed light to the follicle 13 and the papilla 15 causes photothermolysis which provides melanomal disruption, including vaporization of the melanin in the follicle 13 and papilla 15, as well as vacuolation, edema, gas bubbles and protein denaturation. When the absorbed radiation is of sufficient energy level, these effects seriously injure the hair follicle 13 and the papilla 15, thereby damaging the hair germative cells which causes hair regrowth.

However, the presently employed equipment of the prior art is unduly heavy, complicated and expensive; and above all - the operation thereof needs specially trained personnel.

SUMMARY OF THE INVENTION

The present invention is directed to an apparatus for photothermolysis according to the features of appended claim 1.

The reflector may be substantially non-diffusive or may be diffusive.

An optical filter may be arranged with respect to the light source to filter light directed towards the skin. The optical filter may include a cutoff filter in visible and mostly ultraviolet portions of light spectrum.

The light source may be a single flash lamp (e.g., a gas-filled linear flash lamp) or may include a plurality of flash lamps connected electrically in series.

BRIEF DESCRIPTION OF THE DRAWINGS

These and additional constructional features and advantages of the invention will be more readily understood in the light of the ensuing description of embodiments thereof, given by way of example only, with reference to the accompanying drawings wherein:

Fig. 1 is a chart showing the absorption coefficient versus wavelength of radiated light for different substances including melanin;
Fig. 2 shows a cross section of a hair follicle in the dermis;
Fig. 3 is a cross-sectional view of the follicle of Fig. 2 after treatment, showing the damage to the hair follicle;
Fig. 4 is an oscilloscope trace showing the time dependent light pulse response;
Fig. 5A is an electric diagram of capacitor charge and discharge circuit for a single flashlamp;
Fig. 5B is a circuit comprising a pair of flashlamps;
Figs. 6A and 6B are schematic side and front views, respectively, of an incoherent, pulsed light source skin treatment device according to a basic embodiment of the present invention; and
Fig. 7 is a photograph showing skin regions of a patient two months after being treated by the hair removal method of the invention.

DETAILED DESCRIPTION OF EMBODIMENTS

In order to achieve effective photothermolysis based on heat transfer from the hair shaft to the surrounding layers of tissue, it is believed the irradiated light should satisfy the following criteria:

1. Penetration deep enough to reach the hair follicle and papilla;
2. Absorption rate in the hair higher than the absorption of the surrounding tissue; and
3. Pulse duration that should be shorter than the thermal relaxation or cooling time of the treated hair.

In order to meet criteria (1) and (2), the light spectral range should be in the visible to near infrared. A wavelength range of 500 - 1200 nm will penetrate deep enough to reach the hair follicle and at the same time will have significant absorption at the hair shaft, as can be derived from the chart of Fig. 1.

As for condition (3), since the shaft has a much shorter thermal relaxation time (cooling) compared with that of the follicle, the duration of the pulses must be kept shorter than the thermal relaxation time of the shaft to result in ignition/evaporating thereof.

It is well known that the thermal relaxation time of biological objects under temperatures higher than the surrounding tissues is a function, inter alia, of their size and thermal properties. The cooling time, t, of a cylindrical object (such as a hair shaft or follicle) is governed by the

formula $t = \dfrac{d^2}{16\alpha}$

where $\alpha$ is the thermal diffusivity and d is the diameter of the cylindrical object. Hence, the cooling time of a hair follicle is mostly influenced by its diameter. Thus, the cooling time varies by a factor of about 9 between a hair of 100 $\mu$m diameter and a hair of 300 $\mu$m diameter. It is noted that this formula is only approximate and the cooling is also affected by other factors such as thermal conductivity and thermal convection, amongst others.

The diffusivity of hairs is about 0.5 10$^{-7}$ m$^2$/sec. Since both hair shafts and hair follicles are to be destroyed by heat, the pulse duration may be chosen in accordance with shaft diameters that normally measure 50 $\mu$m in diameter. Thus, pulse duration may be significantly less than 3 msec which is the thermal relaxation time of 50 $\mu$m hair shaft.

It is important to understand that the thermal relaxation time is the upper limit for the pulse duration. Shorter pulses can be used to achieve shaft evaporation at lower energy settings.

This is the very reason that the conventional photothermolysis equipment, which utilizes high energy fluence and long pulse width are cumbersome and expensive as already mentioned above.

Since the method of the present invention is based on the principle of energy compressed to short pulses with high peak power, the equipment for practicing it can be of a much smaller size and be readily used by the patients at their homes, as will be explained below.

Operating a flashlamp in pulse mode may include charging a capacitor from a DC power supply during a relatively long duration, e.g., 1 sec, and then followed by abrupt discharging, e.g., less than 0.5 msec as seen in Fig 4. The pulse duration $\underline{T}$ is the result of multiplication of capacitance $\underline{C}$, with the resistance $\underline{R}$ of the light-emitting element, e.g., a xenon lamp, is given by:

$$(\underline{T} = \underline{R} * \underline{C}).$$

The resistance of the flashlamp is practically constant, so the only way to control the pulse width is by adjusting the capacitance. The higher the capacitance the longer the pulse.

The energy stored in the capacitor is

$$J = 0.5CV^2$$

To get an efficient permanent hair removal, one may use high energy fluence and high peak power. Increasing the capacitance also increases the energy fluence. However, in order not to increase the energy fluence too much to prevent burns or chars to the treated tissue, as the capacitance increases the voltage on the capacitor may be controlled so that the fluence does not increase above undesired levels. Reducing the voltage on the capacitor reduces the peak power and reduces the efficacy of the treatment.

Increasing the capacitance increases the volume of the system and the cost of the capacitor and power supply that charges it.

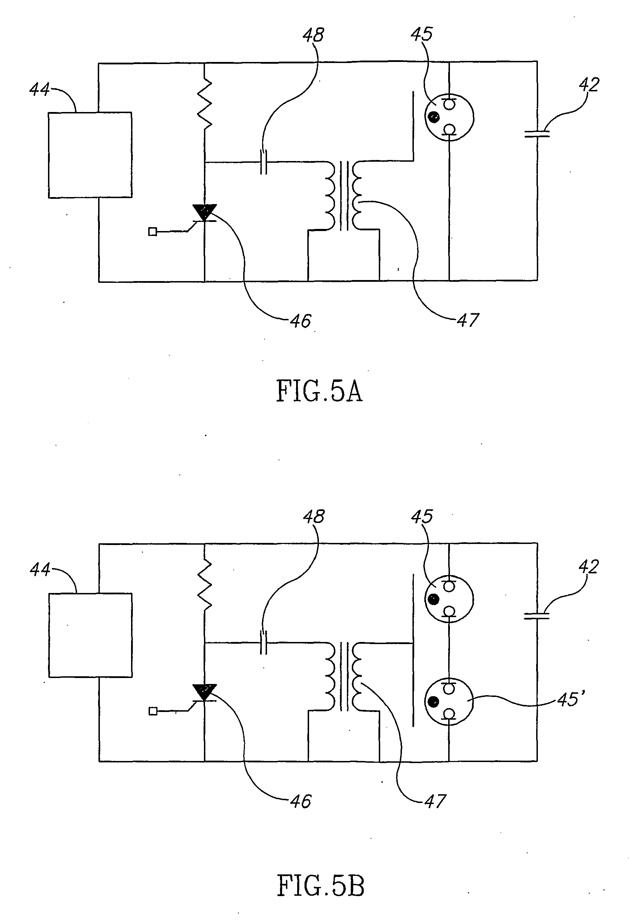

Reference is now made to Fig. 5. In accordance with an embodiment of the invention, a capacitor is used to attain a pulse width of less than 0.5 msec, while at the same time increase the voltage on the capacitor to 1000 V to get both high energy fluence and high peak power, thereby achieving a better treatment efficacy. The energy increases 2:1 with respect to an increase in the voltage, but only decreases 1:1 with respect to a decrease in capacitance. Thus it is advantageous to increase the voltage which more than offsets a decrease in the capacitance. The reduction in the capacitance and the average power of the power supply enables using a system with less volume and lower cost, which are attractive and appropriate for consumer product use.

Based on all the above explained considerations, reference is now made to the remaining figures, which illustrate a photothermolysis system, constructed and operative in accordance with an embodiment of the present invention.

Fig. 5A is an example of a circuit suitable for periodically charging and discharging capacitor 42 through a gas discharge lamp 45, e.g., a xenon flashlamp.

The circuit may further comprise a DC power supply 44, such as a linear or switching power supply. A switch 46 (e.g., SCR switch), transformer 47 and capacitor 48 may build the high voltage ignition to the flashlamp. The invention can be carried out with serial or parallel ignition.

Fig. 5B is the same as in Fig. 5A only that a pair of flashlamps 45 and 45' are provided, connected in series.

Typical values of the components include without limitation: the power supply 44 may be rated for 1000 V, the capacitor 42 may be rated at 300 $\mu$F while capacitor 48 may be rated at 0.1 $\mu$F.

The output of the circuit, namely the characteristic of the light pulses, is illustrated in Fig. 4. It will be noted that the width of the pulse at 1/e of the height of the amplitude is about 0.4 msec.

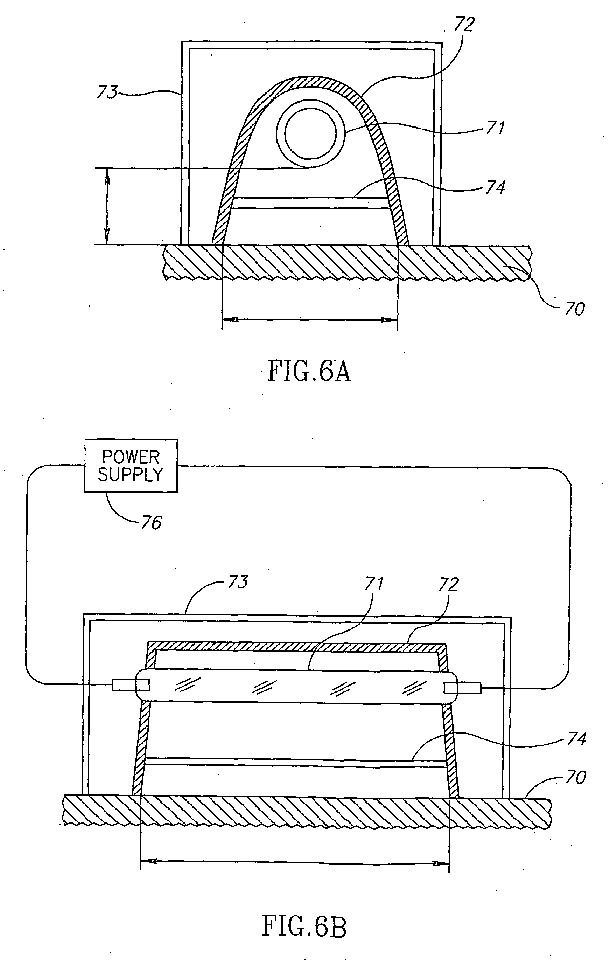

Reference is now made to Figs. 6A and 6B, which illustrate an incoherent, pulsed light source skin treatment device, constructed and operated in accordance with an embodiment of the present invention. The device may include a light source 71 connected to and energized by a power supply 76. A reflector 72 may be arranged with respect to light source 71, which reflects and/or diffuses light emanating from light source 71 towards skin 70 via an opening formed in a housing 73 that at least partially surrounds light source 71 and reflector 72. (The choice of whether reflector 72 is reflective (that is, substantially non-diffusive) or diffusive may be based on the particular application and treatment needs.) An optical filter 74 may be arranged with respect to light source 71, which may filter light, either directly from light source 71 or reflected by reflector 72. Optical filter 74, which may be, without limitation, a neutral density filter, may be mounted near the treatment area to control the spectrum of the light. The filter may be a low cutoff filter in the visible and mostly ultraviolet portions of the spectrum.

Light source 71 may be an incoherent light source, such as but not limited to, a gas-filled linear flash lamp, and may be either a single flash lamp or a plurality of flash lamps connected electrically in series to increase the energy fluence. The flash lamp can be linear, circular or helical, or any other shape. The flash lamp may have an outer enclosure made of bore silica, fuse silica or quartz. The spectrum of light emitted by the gas filled flash lamp may be mostly in the range of 300 to 1200 nm. In accordance with a most preferred embodiment of the invention, the wavelength of the light emitted by light source 71 is in the range of about 350-1200 nm for a duration of about 20-500 $\mu$sec and an energy fluence of about 2-6 J/cm$^2$.

To treat the skin 70, a required light fluence, peak power, pulse width and spectrum wavelength on the skin must be delivered. The light fluence can be achieved with the focusing arrangement of reflector 72 (e.g., reflective or diffusive). The reflector 72 functions as a collimator to the light radiated from the flash lamps, gathering some or most of the light to the treated area and spreading the light evenly over the treatment area.

It is noted that ultrasonic energy by itself has been used in the prior art for treatment of the skin. For example, US Patent Application 2003233103 describes a hand-held ultrasonic device together with an exfoliating fluid to remove unwanted epidermis surface layers, as well as to remove tissue debris and dirt without irritating the skin. However, it is not known to combine ultrasonic energy with flashes of incoherent light.

Thus, in accordance with another embodiment of the invention, the skin may be treated with flashes of incoherent light combined with ultrasonic energy. For example, an ultrasonic transducer 77 (e.g., an oscillating piezoelectric crystal) may be provided to generate ultrasonic energy, such as but not limited to, at a frequency of about 50 KHz to 5 MHz. The ultrasonic transducer 77 may be placed next to the light source 71 or any other suitable place inside housing 73, or may be placed external to housing 73.

It is noted that US Patent Application 2003233103 states that their device is incapable of producing enough energy or heat to substantially exfoliate the epidermis layer of the skin, and that is why they need to use an exfoliating fluid to assist in the resonation and transportation of the energy and heat produced by the ultrasonic waves while also providing healing minerals to help minimize skin irritation. In the present invention, the combination of light energy and ultrasonic energy may synergistically enhance thermolysis of the stratum corneum, epidermis or other skin layers, without the need for an exfoliating fluid. (Of course, an exfoliating liquid can be used in the present invention as well. An example of an exfoliating fluid may include, without limitation, active ingredients aloe vera, yeast, glucosamine, and algae. The glucosamine and yeast may assist in the transfer of energy and heat created by the ultrasonic waves from the ultrasonic transducer 77 to the epidermis. The algae and aloe vera may be used to calm and cool the newly exposed epidermis.)

The ultrasonic energy may also be used to introduce nutrients or other substances transdermally into the skin.

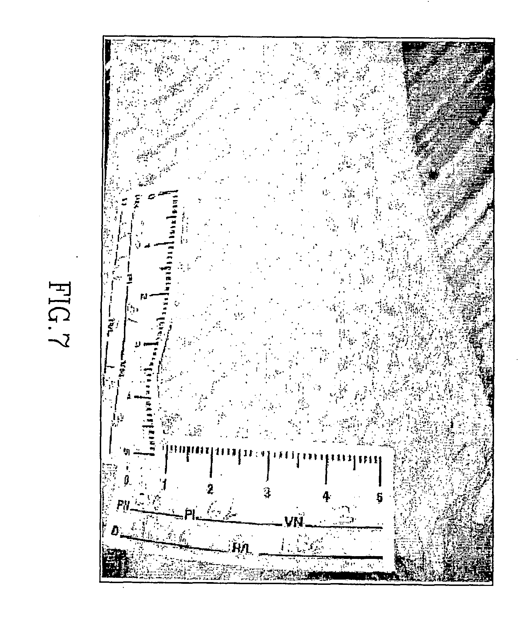

Reference is now made to Fig. 7, which illustrates the results of using the system of the present invention on a human skin with brown hairs and type II Fitzpatrick skin type, two months after the treatment. As can be seen in Fig. 7, hair regrowth was minimal or non-existing, clearly indicating permanent damage to the hair follicles. The experiment used a 300 $\mu$F capacitor charged by 900 V. The light pulse duration measured at 1/e was 0.45 msec and the fluence was 4 J/cm$^2$. The experiment was carried out on both shaved and unshaved areas and the results were the same.

The system was adjusted to a capacitance of 1200 $\mu$F, charged by 350 V and 4 times the pulse width (1.80 msec), but the energy fluence remained at 4 J/cm$^2$. The results were very poor compared with those of the first experiment.

It will be appreciated by persons skilled in the art that the present invention is not limited by what has been particularly shown and described hereinabove. Rather the scope of the present invention includes both combinations and subcombinations of the features described hereinabove as well as modifications and variations thereof which would occur to a person of skill in the art upon reading the foregoing description and which are not in the prior art.

**Claims**

1. Apparatus for skin treatment of the stratum corneum, epidermis or other skin layers by photothermolysis, comprising:

   an incoherent light source (71) connected to and energized by a power supply (76); and a reflector (72) arranged with respect to said light source (71) adapted to direct light emanating from said light source (71) towards skin (70) via an opening formed in a housing (73) that at least partially surrounds said light source (71) and said reflector (72), wherein the light emanating from said light source (71) towards skin (70) comprises flashes of incoherent light of wavelength of about 350-1200 nm for a duration of about 20-500 $\mu$sec and an energy fluence of about 2-6 J/cm$^2$, **characterized by** further comprising an ultrasonic transducer (77) adapted to generate ultrasonic energy having a frequency range of about 50 KHz to 5 MHz towards the skin (70) together with the flashes of incoherent light.

2. The apparatus according to claim 1, wherein said reflector (72) is substantially non-diffusive.

3. The apparatus according to claim 1, wherein said reflector (72) is diffusive.

4. The apparatus according to claim 1, further comprising an optical filter (74) arranged with respect to said light source (71) adapted to filter light directed towards the skin (70).

5. The apparatus according to claim 4 wherein said optical filter (74) comprises a cutoff filter in visible and mostly ultraviolet portions of light spectrum.

6. The apparatus according to claim 1, wherein said light source (71) comprises a single flash lamp.

7. The apparatus according to claim 1, wherein said light source (71) comprises a plurality of flash lamps connected electrically in series.

8. The apparatus according to claim 1, wherein said light source (71) comprises a gas-filled linear flash lamp.

9. The apparatus according to claim 1, wherein said ultrasonic transducer (77) is inside said housing (73).

10. The apparatus according to claim 1, wherein said ultrasonic transducer (77) is external to said housing (73).

**Patentansprüche**

1. Photothermolyse-Vorrichtung zur Hautbehandlung des Stratum corneum, der Epidermis und anderen Hautschichten, Folgendes umfassend:

   eine inkohärente Lichtquelle (71), die an eine Stromversorgung (76) angeschlossen ist und von ihr mit Strom versorgt wird, und ein Reflektor (72), der in Bezug auf die Lichtquelle (71) angeordnet und bereitgestellt wird, um das Licht von der Lichtquelle (71) auf die Haut (70) zu richten, und zwar über eine Öffnung in einem Gehäuse (73), das die Lichtquelle (71) und den Reflektor (72) zumindest teilweise umschließt, wobei das auf die Haut (70) gerichtete Licht aus der Lichtquelle (71) inkohärentes gepulstes Licht mit einer Wellenlänge von 350 - 1 200 nm bei einer Dauer von ca. 20 - 500 μsec sowie eine Energiefluenz von 2 - 6 J/cm$^2$ umfasst, **dadurch gekennzeichnet, dass** sie darüber hinaus einen Ultraschallwandler (77) umfasst, der bereitgestellt ist, um Ultraschallenergie mit einem Frequenzbereich von ca. 50 KHz bis 5 MHz zu generieren, die zusammen mit dem gepulsten inkohärenten Licht auf die Haut (70) gerichtet wird.

2. Vorrichtung nach Anspruch 1, wobei der Reflektor (72) im Wesentlichen nicht streuend ist.

3. Vorrichtung nach Anspruch 1, wobei der Reflektor (72) im Wesentlichen streuend ist.

4. Vorrichtung nach Anspruch 1, darüber hinaus umfassend einen optischen Filter (74), angeordnet in Bezug auf die Lichtquelle (71) und bereitgestellt, um das auf die Haut (70) gerichtete Licht zu filtern.

5. Vorrichtung nach Anspruch 4, wobei besagter optischer Filter (74) einen Cutoff-Filter für die sichtbaren und hauptsächlich ultravioletten Anteile des Lichtspektrums umfasst.

6. Vorrichtung nach Anspruch 1, wobei die Lichtquelle (71) eine einzige Pulslampe umfasst.

7. Vorrichtung nach Anspruch 1, wobei die Lichtquelle (71) mehrere Pulslampen umfasst, die elektrisch hintereinandergeschaltet sind.

8. Vorrichtung nach Anspruch 1, wobei die Lichtquelle (71) eine gasgefüllte Pulsstablampe umfasst.

9. Vorrichtung nach Anspruch 1, wobei der Ultraschallwandler (77) sich innerhalb des Gehäuses (73) befindet.

10. Vorrichtung nach Anspruch 1, wobei der Ultraschallwandler (77) sich außerhalb des Gehäuses (73) befindet.

**Revendications**

1. Appareil pour le traitement cutané de la couche cornée, de l'épiderme ou d'autres couches de la peau par photothermolyse, comprenant :

une source de lumière incohérente (71) reliée à et excitée par une alimentation électrique (76) ; et un réflecteur (72) arrangé par rapport à ladite source de lumière (71) et conçu pour diriger la lumière émanant de ladite source de lumière (71) en direction de la peau (70) via une ouverture pratiquée dans un boîtier (73) qui entoure au moins en partie ladite source de lumière (71) et ledit réflecteur (72), la lumière émanant de ladite source de lumière (71) en direction de la peau (70) comprenant des flashs de lumière incohérente d'une longueur d'onde d'environ 350 à 1200 nm pendant une durée d'environ 20 à 500 μs et une fluence énergétique d'environ 2-6 J/cm$^2$, **caractérisé par le fait qu'**il comprend en outre un capteur ultrasonore (77) conçu pour générer une énergie ultrasonore possédant une plage de fréquence d'environ 50 kHz à 5 MHz en direction de la peau (70) de manière conjointe avec les flashs de lumière incohérente.

2. Appareil selon la revendication 1, dans lequel ledit réflecteur (72) est essentiellement non diffusif.

3. Appareil selon la revendication 1, dans lequel ledit réflecteur (72) est diffusif.

4. Appareil selon la revendication 1, comprenant en outre un filtre optique (74) arrangé par rapport à ladite source de lumière (71) et conçu pour filtrer la lumière dirigée en direction de la peau (70).

5. Appareil selon la revendication 4, dans lequel ledit filtre optique (74) comprend un filtre de coupure dans le spectre visible et dans la plupart des portions ultraviolettes du spectre de lumière.

6. Appareil selon la revendication 1, dans lequel ladite source de lumière (71) comprend une seule lampe flash.

7. Appareil selon la revendication 1, dans lequel ladite source de lumière (71) comprend plusieurs lampes flash montées en série du point de vue électrique.

8. Appareil selon la revendication 1, dans lequel ladite source de lumière (71) comprend une lampe flash linéaire remplie de gaz.

9. Appareil selon la revendication 1, dans lequel ledit capteur ultrasonore (77) est monté à l'intérieur dudit boîtier (73).

10. Appareil selon la revendication 1, dans lequel ledit capteur ultrasonore (77) est situé à l'extérieur dudit boîtier (73).

FIG.1

FIG.2

FIG.3

FIG.4

FIG.5A

FIG.5B

72

73

71

74

70

FIG.6A

POWER
SUPPLY

76

73    71    72

74

70

FIG.6B

FIG.7

# REFERENCES CITED IN THE DESCRIPTION

**Patent documents cited in the description**

- US 5891139 A
- US 5425728 A
- US 5683380 A
- US 5885273 A
- US 6080147 A
- US 20040230260 A1
- US 2003233103 A